# EUROPÄISCHE PATENTSCHRIFT

(11) **EP 2 623 188 B1**
(45) Veröffentlichungstag und Bekanntmachung des Hinweises auf die Patenterteilung: **22.10.2014**
(21) Anmeldenummer: 13000213.2
(22) Anmeldetag: 16.01.2013
(51) Int. Cl.: B01F 1/00, B01F 5/10, B01F 5/04, B01F 15/00, A61M 1/16

(54) **Mischvorrichtung zur Herstellung gebrauchsfertiger medizinischer Spüllösungen insbesondere für die Hämodialyse**
Mixing device for producing ready-to-use medical irrigation solutions in particular for haemodialysis
Dispositif de mélange destiné à fabriquer des solutions de rinçage médicales prêtes à l'emploi, en particulier pour l'hémodialyse

(30) Priorität: 01.02.2012 DE 102012001879
(43) Veröffentlichungstag der Anmeldung: 07.08.2013
(73) Patentinhaber: Völker, Manfred, 63825 Blankenbach (DE)
(72) Erfinder: Völker, Manfred, 63825 Blankenbach (DE)
(74) Vertreter: Flosdorff, Jürgen

(56) Entgegenhaltungen:
- EP-A1- 0 469 487
- WO-A1-00/74833
- DE-A1- 3 844 174

## Beschreibung

Gegenstand der Erfindung ist eine technologisch einfache Mischvorrichtung für die Herstellung gebrauchsfertiger med. Spüllösungen, insbesondere von Konzentraten für die Hämodialyse.

Eventuelle weitere Anwendungsgebiete sind:
- Purisolelösungen für die den Einsatz während einer Blasenoperation oder postoperativen Blasenspülung
- Peritonealösungen für die Bauchfell- Dialysebehandlung
- Spüllösungen für chirurgische Eingriffe
- Spüllösungen für Athroskopie
- (Verteilung fertiger Dialyselösungen)

### Nachteile des Standes der Technik sind:

Die Herstellung ist zeitaufwändig, hoher Energieeinsatz, keine Einhaltung von normativen Vorgaben für fertige Lösungen d.h. unzureichende Genauigkeit der Ionenkonzentration wegen ungenauer Verdünnung bzw. Flüssigkeitszudosierung, dadurch aufwändige hausinterne Kontrolle hinsichtlich Genauigkeit, Mikrobiologie.
Hygiene-/Sterilität
   Unzureichende Mikrobiologie
   Aufwändige Hygienemaßnahmen
Fertiglösungsbehälter
   groß, immobil, fixiert auf fixe Tanks bzw. Behälter
Rohstoffbehälter/Rohstoffe
   Keine Zulassung aller Varianten pulvrig, granuliert, geschlämmt (pastös)
   Rohstoffbeutel oder Rohstoff- Fertiglösungsbeutel sind nicht kombinierbar
Anwendung
   Keine Ideen bzgl. Transfer und Verteilung der Fertiglösung

### Patentanmeldung WO 2010/ 121972

Keine Mischanlage, Komponente einer Mischanlage, lange Mischdauer wegen geringer werdender Konzentrationsdifferenz, keine Aussagen zur Dosierausfallsicherheit, Spülung, Desinfektion des Ansatztanks usw. Mischprozess ist nur erfolgreich bei leicht zu lösenden z.B. granulierten Rohstoffen

### EP 0278 100

On line Produktion von Konzentraten, Mischung erfolgt durch Messung der Konzentration;
Keine Zirkulation zur Verbesserung der Auflösung; keine Bereitstellung des fertigen Konzentrates;
Bevorratung des Rohstoffes in mehreren sep. Behältern.
Herstellung von Bikarbonat; keine ausfallsichere Zudosierung; komplizierter Beutel, keine Zirkulation.

### Patent US 7077956

On line Produktion, keine Zirkulation zur Verbesserung der Auflösung; keine Bereitstellung des fertigen Konzentrates;
Venturi wird für die Entleerung verwendet.

Dokument WO-A1-00/74833 offenbart eine Mischvorrichtung gemäß dem Oberbegriff des Anspruchs 1. Mit der Erfindung werden folgende Verbesserungen angestrebt:
Mischverfahren
   Kurzzeitig
   Energiesparend
   Rückstandsfrei
   Technisch einfach realisierbarer Herstellungsprozess
   Einhaltung von normativen Vorgaben für fertige Lösungen ohne hausinterne Kontrolle
Mischqualität
   Ausfallsichere hochgenaue Zudosierung der Flüssigkeit; unabhängig von der Art der Fertiglösungsbehälter
Hygiene-/Sterilität
   sehr gute Spül- und ggf. Desinfektionsmöglichkeit
   Sterilität , mehrere Filterstufen
Fertiglösungsbehälter
   Mobiler oder stationäre Tanks bzw. Behälter, starre oder flexible Behältnisse
Rohstoffbehälter/Rohstoffe
   Pulvrig, granuliert, geschlämmt (pastös)
Anwendung
   Einfacher Transfer, Beförderung der fertigen Lösung

### Die Verbesserungen sind wie folgt realisiert:

Das Mischgerät enthält einen Rechner mit Ein- und Ausgabeeinheiten, eine Hauptmischleitung bzw. Rezirkulationskreislauf mit einer Zirkulationspumpe, einer ersten Venturipumpe (Venturirohr) und wenigstens einem Ventil und eine Sekundärmischleitung mit einer weiteren Venturipumpe und einem weiteren Ventil.

Die Venturipumpen sind an ihren Ansaugstellen miteinander verbunden, sodass ein bidirektionaler Transport von Flüssigkeit oder Luft-Flüssigkeitsgemisch in den angeschlossenen Rohstoffbehälter möglich ist.

Der flexible beutelartige Rohstoffbehälter ist vorzugsweise ein Wegwerfartikel mit entsprechender transportsicherer Umverpackung und mit pastösen und oder pulvrigen oder granulierten Rohstoffen gefüllt.

Der Rohstoffbehälter wird mittels eines Anschlusses mit Kupplung an die Mischanlage angeschlossen.

Dabei wird über diesen Kupplungsanschluss und die Ventil-Venturipumpeneinheit zum einen Flüssigkeit, aber auch mit großem Vorteil eine Luft-Flüssigkeitsmischung in den Rohstoffbehälter eingesaugt und die Rohstoffe dadurch schnell aufgelöst. Der aufgelöste, hochkonzentrierte Rohstoff wird abgesaugt und in einer Hauptmischleitung zirkuliert, dabei gemischt und vollständig aufgelöst.

Damit kann eine schnelle, kostengünstige, effiziente Aufbereitung von Konzentrat für die Hämodialyse oder eine medizinische Spüllösungen für andere Applikationen hergestellt werden.

Stromabwärts der Kupplung ist eine Spülleitung angeschlossen die bei konnektiertem Rohstoffbehälter nicht durchflossen werden kann. Die Spülleitung wird mit Vorteil an ihrem Ende mit einem Sprühkopf abgeschlossen und mündet dabei so in den Fertiglösungsbehälters ein, dass die Innenoberfläche des Fertiglösungsbehälters nahezu vollständig gereinigt und bei Bedarf desinfiziert werden kann.

Zur Verbesserung der Hygiene und Wiederherstellung der Reinheit nach einem Mischvorgang, oder nach einer längeren betriebsfreien Zeit kann stromabwärts der Spülleitung nach der Kupplungsstelle eine physikalische oder chemische Desinfektionseinrichtung vorgesehen werden.

Dadurch ist ein Eintrag von gefährlichen Desinfiziens während des Mischvorganges nicht möglich.

Um eine rückstandsfreie Entleerung des Fertiglösungsbehälters zu gewährleisten kann an die Mischanlage ein freier Auslauf angeschlossen werden.

Eine wesentliche Komponente der Mischvorrichtung ist die ausfallsichere Zudosierungseinrichtung. Diese Einrichtung überwacht die zugeführte, zur Verdünnung des Rohstoffes benötigte Flüssigkeit, ist mitentscheidend für die Genauigkeit- d.h. die Gebrauchstauglichkeit der Lösung und macht eine weitere zeit- und kostenintensive Überprüfung der fertigen Lösung überflüssig.

Die tankunabhängige Zudosierungseinrichtung besteht in der Basisausführung aus einer Messkammer mit beispielsweise optischen, kapazitiven, oder anderen Messsensoren, die geeignet sind, Flüssigkeit zu detektieren, und mindestens einem angeschlossenen Flussmesser und mindestens einem Ventil.

Darüber hinaus kann die Sensorik für weitere Funktionen wie z.B. auch zur Tankleerkennung eingesetzt werden.

Durch Einsatz dieser Messkammer können mit Vorteil unterschiedliche Fertiglösungsbehälter in Volumen und Ausführung wie z.B. mobile- oder stationäre-, als starre Tanks oder flexible Beutel, Ausführung in den Mischkreis eingebracht werden, weil das Anbringen von Messeinrichtungen an diesen Behältern entfallen kann.

Dabei kann die Zudosierungseinrichtung auch mit dem jeweiligen Fertiglösungsbehälter/ Tank kombiniert werden.

Nachfolgend sind einige Tank-kombinierte Lösungen aufgeführt.

Für eine einfache, d.h. auf ein fixiertes, immer gleiches Füllvolumen ausgelegte Mischvorrichtung genügt ein Füllstandssensor, der nach erfolgter Zudosierung diese beendet. Um den Genauigkeitsanforderungen zu genügen, ist mit Vorteil der Füllstandssensor in einem sich oben teilweise oder ganz verjüngenden Tankbereich angebracht, da insbesondere bei Kunststofftanks durch Montage-, Fertigungs-, Belastungs-, u. Alterungsschwankungen Volumenänderungen nicht auszuschließen sind.

Aufwändigere für unterschiedliche Füllvolumen geeignete, mit dem Fertiglösungsbehälter/ Tank kombinierte Zudosierungseinrichtungen sind z.B. Wäagetechnik, kontinuierliche stetige Füllstandsmessungen wie z.B. Druckmesstechnik, Echolot, oder volumetrische Zudosierungen wie z.B. Bilanzkammern u.a. hier nicht aufgeführte Verfahren.

Alternativ könnten je nach Kosten-, bzw. Sicherheitsaspekten andere als die aufgeführten Ausführungen einer Zudosierungseinrichtung zum Einsatz kommen.

Bei ausreichender Größe des Rohstoffbehälters kann die Zudosierung der Flüssigkeit auch direkt in den Rohstoffbehälter erfolgen, um dadurch die kostenintensive Installation eines Fertiglösungsbehälters einzusparen.

Dadurch besteht die Möglichkeit, die Rohstoffbehälter stationär anzuordnen und die Mischvorrichtung mobil an die Rohstoffbehälter zu bringen bzw. zu konnektieren.

Mit Vorteil wird zur Verbesserung der Hygiene und Sterilität der Flüssigkeit eine Kombination einer RO (Umkehrosomose) mit der Mischanlage vorgesehen.

Darüber hinaus kann die Reinheit der von der RO erzeugten Flüssigkeit über weitere zusätzliche Filterstufen wie z.B. Entionisierung und oder eine Sterilfiltration hinsichtlich Leitfähigkeit und Mikrobiologie so verbessert werden, dass eine gleich gute oder sogar bessere Wasserqualität, wie sie für die Produktion von Arzneimitteln vorgeschrieben ist, erzielt werden kann.

Nach erfolgter Mischung besteht die Möglichkeit, die Fertiglösung über eine weitere Filtereinrichtung mittels Luftdruck oder auch durch Pumpen rückstandfrei zum Einsatzort oder in bereitstehende Bevorratungstanks zu fördern.

Der Transport mobiler Fertiglösungsbehälter zum Einsatzort ist ebenfalls möglich. Bei Verwendung eines luftdruckgeeignetem Behältnisses kann mit großem Vorteil eine kontinuierliche Förderung der Lösung mittels Druckluft erzielt werden. Durch Adaption eines weiteren zusätzlichen Filters, der als Einmalartikel vorzugsweise ein Sterilfilter ist und sich stromabwärts nach dem Behältnis in dem Überleitsystem befindet, ist eine hochsterile Verfügbarkeit der Lösung möglich.

Vorteilhafterweise können bei Verwendung von Druckluft auch die Dichtheit des Systems, insbesondere aber die Dichtheit von eingesetzten stationären Sterilfiltern überprüft werden.

Falls eine temperierte Flüssigkeit zur Anwendung kommen soll, kann eine optional zur Verfügung stehende Heizung eingesetzt werden.

Mit dieser Mischvorrichtung sind Ausführungsvarianten jeweiliger landes- als dialysestationsspezifischer Vorgaben möglich.

So ist es vorstellbar, dass in kleinen HD Stationen eher mobile Behältnisse- und in größeren Stationen eher stationäre Fertiglösungsbehältnissen zum Einsatz kommen.

Da kein Rohstoffwechselbehälter zum Einsatz kommt entfallen teure Transportretouren.

Die Mischqualität ist rückstandsfrei, die Vorrichtung arbeitet energiesparend. Da sowohl der Rohstoff validiert-, also ein medizinisches Produkt ist, als auch die zugeführte Flüssigkeit hinsichtlich Reinheit und Exaktheit des Volumens den Genauigkeitsanforderungen entsprechen, entfallen aufwändige hausinterne Kontrollen.

Es ist ebenso möglich, hygienisch weniger anspruchsvolle Lösungen, oder durch Hinzunahme weiterer Filterstufen hochsterile Medikamente herzustellen. Aufheizen zum schnelleren Auflösen und oder für eine temperierten Anwendung bzw. Warmhalten der Lösung, sowie einfachste Verteilung mittels Luftdruck runden die Erfindung ab.

Nachfolgend werden Ausführungsformen der Erfindung mit Bezug auf die Zeichnungen näher beschrieben. Diese zeigen.
- Fig.1: Vorrichtung zum Mischen eines Rohstoffes in einem Beutel mit einem Anschluss und Fertiglösungsbehälter
- Fig.1.1: Vorrichtung zum Mischen eines Rohstoffes in einem Beutel mit einem Anschluss ohne Fertiglösungsbehälter
- Fig.2: Vorrichtung zum Mischen eines Rohstoffes in einem Beutel mit einem Anschluss und mobilem Fertiglösungsbehälter und steriler Flüssigkeitszuführung
- Fig.2.1: Vorrichtung zum Mischen eines Rohstoffes in einem Beutel mit einem Anschluss und mobilem Fertiglösungsbehälter und steriler Flüssigkeitszuführung und hochgenauer Zudosierung
- Fig.2.2: Vorrichtung zum Mischen eines Rohstoffes in einem Beutel mit einem Anschluss und mobilem Fertiglösungsbehälter, steriler Flüssigkeitszuführung, hochgenauer Zudosierung und zusätzlicher Sterilfiltration

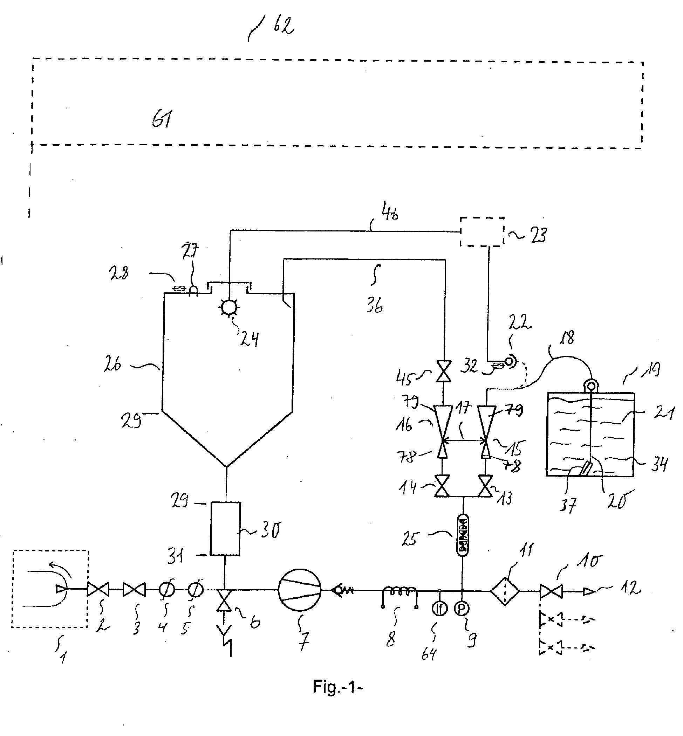

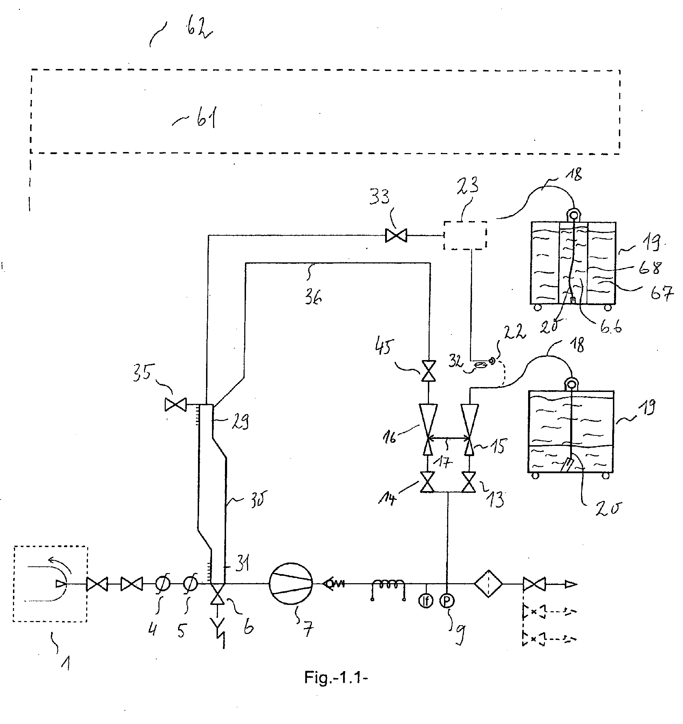

Dabei zeigt Figur 1 die Flüssigkeitszuführung (1), die ausfallsichere Zudosierung die aus folgenden Komponenten Betriebsventil (2), Schutzventil (3), Betriebsflussmesser (4), Schutzsystemflussmesser (5) und Messkammer (30) mit den Niveau Sensoren (31/29) besteht. Vorteilhafterweise werden die Ventile (2/3) in unterschiedlichen Ausführungen, z. B. Membranventil und/oder Servoventil eingesetzt, um einen Ausfall bzw. eine fehlerhafte Zudosierung zu verhindern. Die beiden Flussmesser (4/5) werden durch den Rechner (61) der Mischanlage (62) überprüft und durch das Zulaufvolumen in die Messkammer (30) und die Niveau Sensoren (29/31) verifiziert. In der einfachsten Ausführung kann die ausfallsichere Zudosierung aus der Messkammer (30), den Niveausensoren (29/31) und mindestens einem Flussmesser und mindestens einem Ventil bestehen.

Der Fertiglösungsbehälter (Ansatzbehälter) (26) wird über die Messkammer (30) gefüllt bis das erforderliche Volumen erreicht ist. Die Entlüftung des Fertiglösungsbehälters (26) erfolgt über den Entlüftungsfilter (27). Ein Überlauf kann mit Leckagemelder (28) detektiert werden.

Eine Entleerungsmöglichkeit des Fertiglösungsbehälters (26) erfolgt über Abflussventil (6), welches aus Gründen der Rückkontamination stromabwärts in einen freien Auslauf mündet. Dieser Weg wird vorzugsweise nach einem Spülvorgang oder als Verwurfsmöglichkeit bei fehlerhafter Fertiglösung gewählt. Vorteilhafterweise wird zu Beginn des Füllprozesses ein Teil des Zuflusses über Abflussventil (6) zur Verhinderung eines Keimeintrages verworfen.

Der Zufluss zum Fertiglösungsbehälter (26) kann ebenfalls über die Flussmesser und Messkammer von oben in den Behälter erfolgen.

Zu Beginn des Mischprozesses fördert die Pumpe (7) einen Teil der Flüssigkeit aus Fertiglösungsbehälter (26) über die optionale Heizung (8) und den optionalen statischen Mischer (25) über Sekundärmischventil (13), Sekundärventuri (15) und Anschlussschlauch (18) in den angeschlossenen Rohstoffbehälter-/beutel(19).

Die Spülleitung (46) mit der optionalen Desinfektion (23) und dem Reinigungssprühkopf (24) bleiben über die Kupplungsstelle (22) offen.

Nach erfolgter Zudosierung in Rohstoffbehälter (19) schließt Ventil (13). Hauptmischventil (14) und Füllhilfsventil (45) werden geöffnet und die Flüssigkeit zirkuliert über Mischventuri (16), Hauptmischleitung (36) und Tank (26). Dabei wird Flüssigkeitsrohstoffgemisch über Sauglanze (20), Venturi (15) und Ansaugstelle (17) in die Mischventuri (16) eingesaugt und über die Hauptmischleitung (36) zirkuliert und vermischt.

Danach erfolgt ein erneuter Flüssigkeitseintrag über Ventil (13) und Venturi (15) in Rohstoffbehälter (19). Bei geöffnetem Ventil (45) und geschlossenen Ventil (14) wird dabei Luft durch die Hauptmischleitung (36) und Venturi (16) im Gegenstrom mittels Venturi (15) angesaugt und als Luft-Flüssigkeitsgemisch in Rohstoffbehälter (19) eingebracht. Die eingetragene Luft beschleunigt die Auflösung der im Rohstoffbehälter (19) befindlichen Rohstoffe (21).

Die Restentleerung des Rohstoffbehälters (19) erfolgt durch den Treibstrom über Mischventuri (16), diese saugt dabei das Flüssigkeits-/Rohstoffgemisch (34) in Fertiglösungsbehälter (26). Dabei kann die Leererkennung über einen hier nicht dargestellten Sensor an geeigneter Stelle überwacht werden.

Zur Drucküberwachung des Rohstoffbehälters (19) dient Drucksensor (9) der auch an anderer, hier nicht dargestellter Stelle des Hydraulikkreislaufes angebracht werden kann. Dabei kann Drucksensor (9) die Dichtheit der angeschlossenen Komponenten wie z.B. Transferfilter (11), Transferventil (10) mit überprüfen.

Nach erfolgter Entleerung des Rohstoffbehälters (19) wird Schlauch (18) an Kupplungsstelle (22) -an ihre Parkstation- konnektiert. Optional gibt es hierfür einen Meldekontakt (32).

Der entleerte Rohstoffbehälter (19) kann ein Einwegbehälter sein.

Die Fertiglösung wird aus dem Fertiglösungstank (26) mittels Pumpe (7) und Transferventil (10), Transferfilter (11) zum Anwendungsort oder in bereitgestellte Tanks gepumpt.

Die Spülung der Mischanlage (61) erfolgt im Wechsel so, dass alle Leitung (36/46) durchflossen werden, wobei der Reinigungssprühkopf (24) so angebracht ist, dass die gesamte Innenoberfläche des Fertiglösungsbehälters (26) besprüht werden kann.

Zur Verbesserung der Reinigungseffektivität kann optional eine Reinigungs-/Desinfektionsvorrichtung (23) in die Leitung (46) zum Fertigungslösungsbehälter (26) eingebaut werden. Eine unerwünschte Desinfektion während des Mischvorgangs ist wegen der offenen Kupplungsstelle (22) nicht möglich. Zur Überprüfung der Konzentration, sowohl der fertigen Mischung als auch einer Desinfektionslösung kann Leitfähigkeitsmessung (64) eingesetzt werden.

Figur 1.1 zeigt eine Mischvorrichtung ohne Ansatztank. Dabei werden die Flussmesser (4/5) über Messkammer (30) verifiziert. Die Füllung des Rohstoffbehälters (19) erfolgt über Leitung (36), Ventil (45) über Venturiverbindung (17) in Rohstoffbehälter (19), der nur teilweise mit Rohstoff (21) gefüllt ist.

Der Lufteintrag über Ansaugung der Venturi (15), zur Beschleunigung der Rohstoffauflösung, erfolgt entweder über das geöffnete Ventil (33) und Kupplungsstelle (22) oder das optionale Entlüftungsventil (35). Dabei bleibt Ventil (14) geschlossen. Der Mischvorgang d.h. die wechselweise Füllung und Absaugung des Rohstoffbehälter (19) erfolgt wie bereits in Figur 1 beschrieben. Zur Verbesserung der Mischeffizienz kann dabei ein statischer Mischer (25) wie in Fig. 1 dargestellt mit eingesetzt werden. Vorteilhafterweise ist hierbei der Rohstoffbehälter (19) mobil und kann nach durchgeführtem Mischvorgang als Fertiglösungsbehälter zum Einsatzort gebracht werden. Ebenso ist ein Transfer wie in Figur 1 beschrieben möglich.

Die Messkammer ist in drei Abschnitte mit einem mittleren großvolumigeren Teil und zwei Strecken mit kleineren Querschnitten gegliedert. Die beiden Endabschnitte dienen dabei der feineren Auflösung und dort kann der Füllstand entweder als transparente Messstrecke optisch oder auch kapazitive mittels Niveausensoren (29/31) bzw. mit anderer Sensortechnik durch den Rechner (61) der Mischanlage (62) ermittelt werden. Der oder auch die von den Flussmessern abgegebenen Werte werden mit dem als bekannt vorausgesetzten Volumen der Messkammer (30) verglichen bzw. verifiziert. Dabei kann die Messkammer (30) auch konstruktiv andere geometrische Formen aufweisen als in Fig. 1 dargestellt.

Figur 1.1 zeigt auch eine schematische Darstellung eines Zweikammerbeutels (19) für Rohstoffe die aufgrund ihrer chemischen Reaktionen in getrennte Behälter unterzubringen sind, z. B. für Glucose und schwerlösliche Salze hoher Konzentration in Verbindung mit Essigsäure. Vorgesehen ist beispielsweise die Glucose in Kammer (66) und schwerlösliche Salze hoher Konzentration in Verbindung mit Essigsäure in Kammer (67) unterzubringen.

Zu Beginn des Mischvorganges wird zunächst mittels Pumpendruck der Pumpe (7) bei geschlossenem Ventil (45) Flüssigkeit über Schlauch (18) in Kammer (66) eingebracht bis die Peelnaht (68) aufreißt und die beiden Kammern (66/67) verbunden sind, um danach den Mischprozess wie bereits beschrieben durchzuführen. Die Lage der Peelnaht ist dabei nicht auf die angedeutete Stelle begrenzt.

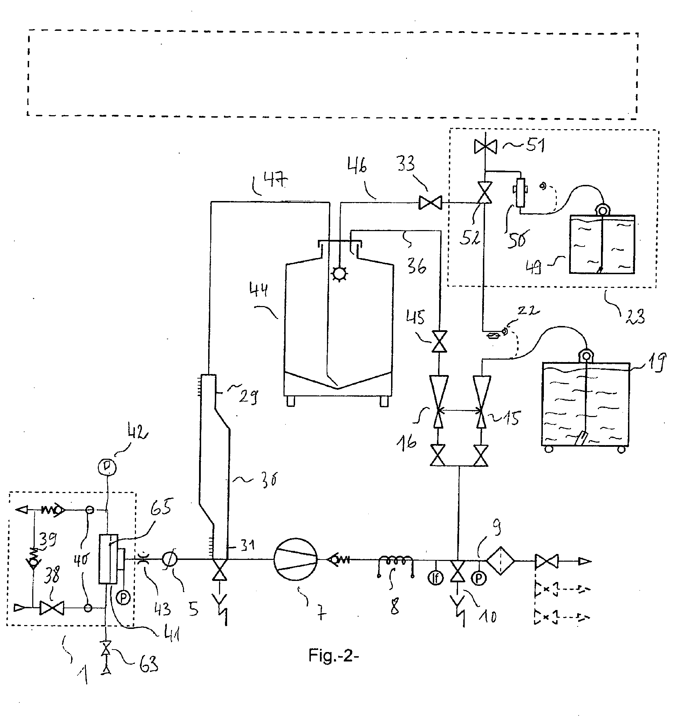

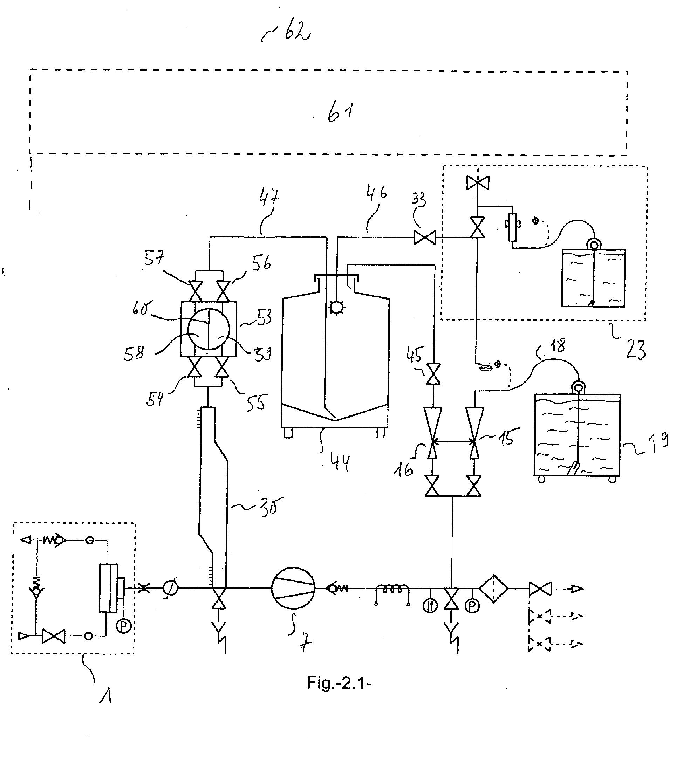

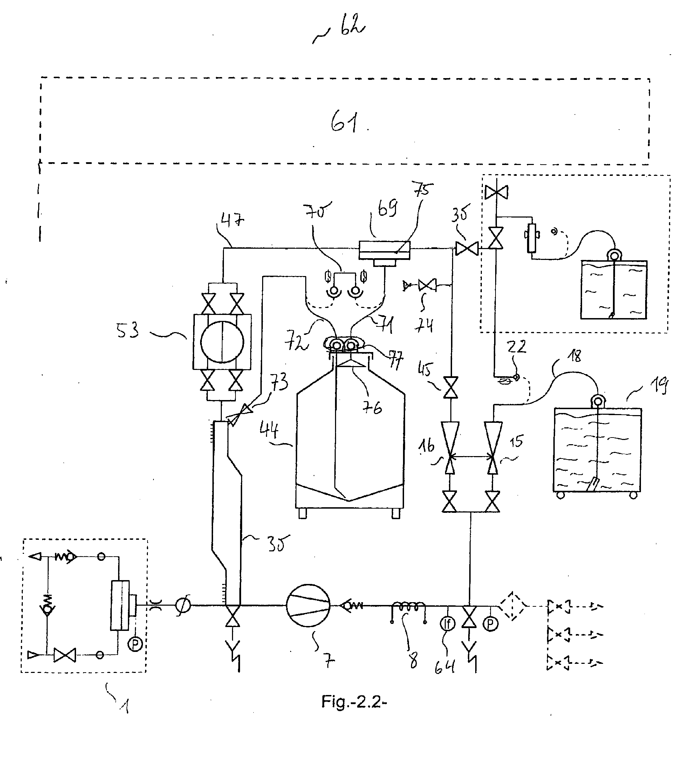

Figur 2 zeigt den Einsatz einer Mischvorrichtung mit einem sterilen Flüssigkeitsversorgungsanschluß (1), wobei die Flüssigkeit einer vorgeschalteten hier nur teilweise dargestellten Umkehrosmose (RO) über Sterilfilter (41) zur Messkammer (30) eingebracht wird.

Dabei dienen die Flussmesser (40), die als integraler Bestandteil einer Umkehrosmose-Anlage symbolisch dargestellt sind, als Betriebsflussmesser und Flussmesser (5) als Schutzsystemflussmesser. Ventil (38), ebenfalls integraler Bestandteil einer Umkehrosmose-Anlage, dient als Schutzventil für fehlerhafte Zudosierung, wobei in diesem Fall auch die Umkehrosmose-Anlage abgeschaltet werden kann. Die Lage der dargestellten Flusssensoren ist innerhalb der RO auch an anderen dem funktionellen Zweck dienenden Stellen möglich. Anstelle einer RO ist auch der Einsatz einer in einem Tank bereitgestellten Flüssigkeit mit Druckerhöhungsanlage möglich.

Zur Validierung des Sterilfilters (41) kann mittels Luftanschluss (63) Druckluft auf die Primärseite des Sterilfilters gefördert und die Flüssigkeit aus dem Primärraum verdrängt werden. Bei einer intakten Sterilfiltermembran (65) kann kein Druckabfall durch Drucksensor (42) registriert werden, weil die intakte Membrane (65) eine luftundurchlässige Funktion hat.

Der mobile Fertiglösungsbehälter (44) kann dabei sowohl aus flexiblem Kunststoff als auch starrem Material, z.B. aus Glas gefertigt sein. Nach erfolgter Mischung kann dieses Behältnis (44) einfach zum Einsatzort gerollt werden.

Figur 2 zeigt außerdem eine chemische Dosier-/Reinigungsvorrichtung (23). Bei konnektierter Kupplung (22) an die Spülleitung (46), geöffnetem Ventil (52), geschlossenem Ventil (51) kann mittels Venturi (16) im Gegenstrom über Venturi (15) Desinfektions-/Reinigungsmittel aus Kanister (49) angesaugt werden, bis der Leitfähigkeits-Temperatur-Sensor (64) die gewünschte Zielleitfähigkeit registriert. Die Niveauüberwachungskammer (50) kann sowohl als Leererkennung für Kanister (49) als auch als Schutzsystem für ungewünschtes Ansaugen fungieren, da Ventil (51) im Ruhezustand offen ist muss niveauüberwachte Kammer (50) entleert sein.

Die Desinfektion/Reinigung erfolgt wechselweise über Leitung (36, 46, 47) der Mischanlage. Nach einer Desinfektion wird das Desinfektionsmittel über Ventil (10) ausgespült.

Mit Einrichtung (23) ist auch die sichere Zudosierung kritischer flüssiger Substanzen wie z.B. Essigsäure für die Herstellung von HD Konzentraten möglich.

Anstelle einer chemischen Reinigungsvorrichtung (23) wäre auch eine die Desinfektion betreffende, gleichwertige physikalische Reinigungsvorrichtung (23) mit einer Ozonzelle möglich.

Ebenso ist eine thermische Desinfektion oder eine Kombination thermisch und physikalisch mittels Heizung (8) möglich.

Figur 2.1 zeigt zur Verbesserung der Dosiergenauigkeit eine Bilanzkammer (53), die in die Füll- bzw. Abflussleitung (47) eingebracht ist, dabei kann die Messkammer (30) entfallen. Die Füllung der Kammer (58) erfolgt durch den Vordruck der Flüssigkeitsquelle (1) und beispielsweise geöffnetem Füllventil (54), geschlossenem Entleerventil (57), dabei bleibt Füllventil (55) geschlossen und Entleerventil (56) geöffnet. Die flexible Membran (60) verdrängt dabei die Flüssigkeit aus Kammer (59) in den angeschlossen Fertiglösungsbehälter (44). Durch wechselweise Schaltung der Ventile (54, 55, 56, 57) werden die Kammern (58, 59) gefüllt und entleert. Durch das konstruktiv vorgegebene Kammervolumen kann eine genaue zu Dosierung erfolgen. Die Zirkulation aus Fertiglösungsbehälter (44) mittels Pumpe (7) wird bei geöffneten Dosierkammerventilen (54, 55, 56, 57) durchgeführt.

Figur 2.2 zeigt einen zusätzlichen Füllfilter (69), der auch als Sterilfilter ausgebildet werden kann. Die zugeführte Flüssigkeit wird über Filtermembran (75) und Füllanschluss (71) flüssigkeitsberuhigt über Einlaufkonus (76) in Füllbehälter (44) eingebracht. Die Zirkulation wird über Leitung (72), Ventil (73) und Pumpe (7) durchgeführt.

Mittels Heizung (8) und Messeinrichtung (64) kann die Fertiglösung auf eine gewünschte Temperatur und Konzentration eingestellt werden. Dabei bietet der Rechner (61) der Mischanlage (62) auch die Möglichkeit einer Registrierung der verwendeten Rohstoffe bzw. des erzeugten Produkts beispielsweise mittels Barcodeleser und angeschlossenen Drucker. Die Leitungen (71, 72) können dabei aus Gründen der einfachen Handhabung zu einem Zwillingsschlauch mit einer Kupplung (77) zusammengefasst werden. Nach Abschluss der Mischung und anschließender Leerung des Rohstoffbehälters (19) wird dieser entsorgt. Schlauch (18) wird auf Kupplungsstelle (22) und Kupplung (77) wird auf Parkstation (70) zurückgesteckt. Die Mischanlage (62) ist danach wieder betriebsbereit und kann gespült bzw. desinfiziert werden. Der Fertiglösungsbehälter (44) kann zum Einsatzort gebracht und dort vorzugsweise mittels Luftdruck an Kupplungsstelle (71) und einem Einmalartikel mit Filter an Kupplungsstelle (72) patientennah verarbeitet werden.

Es wird betont, dass die Erfindung nicht auf die beschriebenen und dargestellten Ausführungsformen beschränkt ist. Vielmehr sind alle offenbarten Merkmale der Ausführungsformen auch einzeln untereinander anders als oben beschrieben miteinander kombinierbar.

**Legende**

| | |
|---|---|
| 1. | Flüssigkeitsversorgungsanschluss |
| 2. | Betriebsventil |
| 3. | Schutzventil |
| 4. | Betriebsflussmesser |
| 5. | Schutzsystemflussmesser |
| 6. | Abflussventil |
| 7. | Zirkulationspumpe |
| 8. | Optionale Heizung |
| 9. | Drucksensor |
| 10. | Transferventil |
| 11. | Transferfilter |
| 12. | (Transferanschluss) |
| 13. | Sekundärmischventil |
| 14. | Hauptmischventil |
| 15. | Sekundärventuri |
| 16. | Misch-/Belüftungsventuri |
| 17. | Verbindung-Ansaugstellen Venturi |
| 18. | Anschlussschlauch |
| 19. | Rohstoffbehälter-/ Beutel |
| 20. | Sauglanze mit Ansaugspitze |
| 21. | Rohstoff |
| 22. | Kupplungsstelle |
| 23. | Desinfektion (opt.) |
| 24. | Reinigungssprühkopf |
| 25. | Statischer Mischer (opt.) |
| 26. | Fertiglösungsbehälter (Ansatzbehälter mit Restentleerung) |
| 27. | Entlüftungsfilter |
| 28. | Überlaufleckage |
| 29. | Niveausensor |
| 30. | Messkammer/ (Ansatzbehälter) |
| 31. | Niveausensor |
| 32. | Meldekontakt |
| 33. | Sperrventil-/Belüftungsventil |
| 34. | Flüssigkeits- Rohstoffgemisch |
| 35. | Belüftungsventil |
| 36. | Hauptmischleitung |
| 37. | Sieb |
| 38. | Reinstwasserversorgungssperrventil |
| 39. | Überströmventil |
| 40. | Betriebssystemflussmesser |
| 41. | Filter/Sterilfilter |
| 42. | Drucksensor |
| 43. | Konstantflussdrossel (option) |
| 44. | Mobiler Fertiglösungsbehälter |
| 45. | Füllhilfsventil Rohstoffbehälter |
| 46. | Spülleitung |
| 47. | Füll- u. Abflussleitung Fertiglösungsbehälter |
| 48. | |
| 49. | Desinfektionskanister |
| 50. | Niveauüberwachungskammer Desinfektionskanister |
| 51. | Belüftungsventil |
| 52. | Desinfektionsfreigabeventil |
| 53. | Dosierkammer (Bilanzkammer) |
| 54. | Füllventil |
| 55. | Füllventil |
| 56. | Entleerventil |
| 57. | Entleerventil |
| 58. | Dosierkammer 1 |
| 59. | Dosierkammer 2 |
| 60. | Membrane |
| 61. | Rechner der Mischanlage |
| 62. | Mischanlage |
| 63. | Druckluftzufuhr |
| 64. | Leitfähigkeitstemperaturmessung (opt.) |
| 65. | Sterilfiltermembrane |
| 66. | Rohstoffkammer 1 |
| 67. | Rohstoffkammer 2 |
| 68. | Peelnaht |
| 69. | Füllfilter |
| 70. | Parkposition |
| 71. | Füllanschluss |
| 72. | Entleeranschluss |
| 73. | Absaugventil |
| 74. | Druckluftventil |
| 75. | Filtermembrane |
| 76. | Einlaufkonus |
| 77. | Fertiglösungskupplung |
| 78. | Konvergenzkammer eines Venturirohrs |
| 79. | Divergenzkammer eines Venturirohrs |

## Patentansprüche

1. Mischvorrichtung zur Herstellung gebrauchsfertiger medizinischer Spüllösungen, insbesondere für die Hämodialyse, mit einer Reinstwasserquelle (1), die über eine Zulaufleitung mit einem Rezirkulationskreislauf (36) verbunden ist, in den eine Pumpe (7) eingeschaltet ist, mit einem Rechner (61) und
mit einer Anschlussleitung (18), die mit einem Rohstoffbehälter (19) koppelbar ist, der vor Beginn des Mischvorgangs pulverige und/oder granulierte und/oder geschlämmte Rohstoffe enthält, die mit dem Reinstwasser gemischt werden sollen,
wobei in den Rezirkulationskreislauf nacheinander ein Hauptmischventil (14), ein Misch-/Belüftungsventurirohr (16) mit einer Konvergenzkammer (78) und einer Divergenzkammer (79) und ein Füllhilfsventil (45) eingeschaltet sind,
**dadurch gekennzeichnet, dass** parallel dazu ein Sekundärmischventil (13) und ein Sekundarventurirohr (15) mit einer Konvergenzkammer (78) und einer Divergenzkammer (79) in die von dem Rezirkulationskreislauf (36) abgezweigte Anschlussleitung (18) eingeschaltet sind, und
**dass** die Ansaugstellen der Venturirohre (15,16) durch eine Leitung (17) verbunden sind.

2. Mischvorrichtung nach Anspruch 1,
**dadurch gekennzeichnet,**
**dass** in den Rezirkulationskreislauf ein Fertiglösungsbehälter(26) eingeschaltet ist, wobei eine Hauptmischleitung (36) des Rezirkulationskreislaufs oben in den Fertiglösungsbehälter einmündet.

3. Mischvorrichtung nach Anspruch 2,
**dadurch gekennzeichnet,**
**dass** oben in den Fertiglösungsbehälter (26) eine Spülleitung (46) einmündet, die anderenends mit einer Kupplung (22) versehen ist, die mit der bevorzugt schlauchförmigen Anschlussleitung (18) koppelbar ist.

4. Mischvorrichtung nach Anspruch 3,
**dadurch gekennzeichnet,**
**dass** die Spülleitung (46) mittig in den Fertiglösungsbehälter einmündet und mit einem Sprühkopf (24) versehen ist.

5. Mischvorrichtung nach den Ansprüchen 1 bis 4,
**dadurch gekennzeichnet,**
**dass** von dem Rezirkulationskreislauf eine Transferleitung mit einem Transferventil (10) und vorzugsweise einem Transferfilter (11) abzweigt.

6. Mischvorrichtung nach Anspruch 1 bis 5,
**dadurch gekennzeichnet,**
**dass** in die Zulaufleitung oder den Rezirkulationskreislauf wenigstens ein mit dem Rechner verbundener Flussmesser (4,5) eingeschaltet ist und dass stromabwärts davon eine volumetrische Messkammer (30) angeordnet ist, in der der Füllstand der Messkammer (30) ermittelt wird, wobei der ermittelte Wert durch den Rechner mit dem Messwert des wenigstens einen Flussmessers verglichen und dessen Messwert erforderlichenfalls korrigiert wird.

7. Mischanlage nach Anspruch 6,
**dadurch gekennzeichnet,**
**dass** zwei Flussmesser (4,5) hintereinander angeordnet sind, die als Betriebsflussmesser (4) und Schutzsystemflussmesser (5) redundant arbeiten und von dem Rechner der Mischvorrichtung überwacht werden.

8. Mischvorrichtung nach den Ansprüchen 1 bis 7,
**dadurch gekennzeichnet,**
**dass** der Rohstoffbehälter (19) ein Einwegbehälter ist.

9. Mischvorrichtung nach den Ansprüchen 1 bis 8,
**dadurch gekennzeichnet,**
**dass** eine Sauglanze (20) zum Bodenbereich des Rohstoffbehälters (19) führt, durch die Flüssigkeitsrohstoffgemisch in den Rezirkulationskreislauf gesaugt werden kann.

10. Mischvorrichtung nach den Ansprüchen 1 bis 9,
**dadurch gekennzeichnet,**
**dass** eine Dosierkammer Bilanzkammer (53) in die zum Fertiglösungsbehälter (44) führenden Füll/Abflussleitung (47) des Rezirkulationskreislaufs eingeschaltet ist.

## Claims

1. A mixing device for producing ready-to-use medicinal irrigation solutions, particularly for haemodialysis, with an ultrapure water source (1), which is connected by means of a feed line to a recirculation circuit (36), connected into which there is a pump (7), a data processor (61) and a connecting line (18), which may be coupled to a raw material container (19), which, before the beginning of the mixing process, contains pulverulent and/or granulated and/or slurried raw materials, which are to be mixed with the ultrapure water, wherein connected successively into the recirculation circuit there are a main mixing valve (14), a mixing-aeration venturi tube (16) with a convergence chamber (78) and a divergence chamber (79) and an auxiliary filling valve (45), **characterised in that** connected in parallel therewith into the connecting line (18) branched off from the recirculation circuit (36) there are a secondary mixing valve (13) and a secondary venture tube (15) with a convergence chamber 78) and divergence chamber (79) and that the suction points of the venturi tubes (15,16) are connected by a line (17).

2. A mixing device as claimed in claim 1, **characterised in that** connected into the recirculation circuit there is a finished solution container (26), wherein a main mixing line (36) of the recirculation circuit discharges into the top of the finished solution container.

3. A mixing device as claimed in claim 2, **characterised in that** discharging into the top of the finished solution container (26) there is an irrigation line (46), which is provided at its other end with a coupling (22) which may be coupled to the preferably hose-shaped connecting line (18).

4. A mixing device as claimed in claim 3, **characterised in that** the irrigation line (46) discharges centrally into the finished solution container and is provided with a spray head (24).

5. A mixing device as claimed in claims 1 to 4, **characterised in that** branching off from the recirculation circuit there is a transfer line with a transfer valve (10) and preferably a transfer filter (11).

6. A mixing device as claimed in claims 1 to 5, **characterised in that** connected into the feed line or the recirculation circuit there is at least one flow meter (4, 5) connected to the data processor and that arranged downstream thereof there is a volumetric measuring chamber (30), in which the filling level of the measuring chamber (30) is determined, wherein the determined value is compared by the data processor with the measured value of the at least one flow meter and its measured value is corrected, if necessary.

7. A mixing device as claimed in claim 6, **characterised in that** two flow meters (4, 5) are arranged one after the other, which operate in a redundant manner as an operational flow meter (4) and a protective system flow meter (5) and are monitored by the data processor of the mixing device.

8. A mixing device as claimed in claims 1 to 7, **characterised in that** the raw material container (19) is a disposable container.

9. A mixing device as claimed in claims 1 to 8, **characterised in that** a suction lance (20) extends to the base region of the raw material container (19), through which the liquid raw material mixture can be sucked into the recirculation circuit.

10. A mixing device as claimed in claims 1 to 9, **characterised in that** a metering chamber-balancing chamber (53) is connected into the filling/discharge line (47) of the recirculation circuit leading to the finished solution container (44).

## Revendications

1. Dispositif de mélange pour la production de solutions de rinçage médicales prêtes à l'emploi, en particulier pour l'hémodialyse, comportant une source d'eau ultra-pure (1) qui est reliée par une conduite d'alimentation à un circuit de recirculation (36) dans lequel une pompe (7) est activée, comportant un ordinateur (61) et
une conduite de raccordement (18) qui peut être couplée à un contenant de matière première (19), qui contient avant le début du processus de mélange, des matières premières pulvérulentes et/ou granulées et/ou en suspension, qui doivent être mélangées avec l'eau ultra-pure,
dans lequel, dans le circuit de recirculation sont activés consécutivement une vanne de mélange principale (14), un tube Venturi de mélange/aération (16) doté d'une chambre de convergence (78) et d'une chambre de divergence (79) et une vanne de remplissage auxiliaire (45),
**caractérisé en ce que**
parallèlement sont activés une vanne de mélange secondaire (13) et un tube Venturi secondaire (15) doté d'une chambre de convergence (78) et d'une chambre de divergence (79) dans la conduite de raccordement (18) se ramifiant à partir du circuit de recirculation (36), et
les sites d'aspirations des tubes Venturi (15, 16) sont reliés par une conduite (17).

2. Dispositif de mélange selon la revendication 1,
**caractérisé en ce que**
dans le circuit de recirculation, un contenant de solution prête (26) est activé, une conduite de mélange principale (36) du circuit de recirculation débouchant dans le contenant de solution prête.

3. Dispositif de mélange selon la revendication 2,
**caractérisé en ce que**
au niveau supérieur, dans le contenant de solution prête (26), une conduite de rinçage (46) débouche, qui peut être dotée à l'autre extrémité, d'un couplage (22) qui peut être couplé à la conduite de raccordement, de préférence sous la forme d'un tuyau (18).

4. Dispositif de mélange selon la revendication 3,
**caractérisé en ce que**
la conduite de rinçage (46) débouche au milieu du contenant de solution prête et est dotée d'une tête de pulvérisation (24).

5. Dispositif de mélange selon les revendications 1 à 4,
**caractérisé en ce que**
à partir du circuit de recirculation, se ramifie une conduite de transfert dotée d'une vanne de transfert (10) et de préférence, d'un filtre de transfert (11).

6. Dispositif de mélange selon les revendications 1 à 5,
**caractérisé en ce que**
dans la conduite d'alimentation ou le circuit de recirculation, est activé au moins un débitmètre (4, 5) relié à l'ordinateur et **en ce que**, en aval de celui-ci, une chambre de mesure volumétrique (30) est disposée, dans laquelle le niveau de remplissage de la chambre de mesure (30) est déterminé, la valeur déterminée étant comparée par l'ordinateur à la valeur de mesure de l'au moins un débitmètre, et dont la valeur de mesure est corrigée si nécessaire.

7. Installation de mélange selon la revendication 6,
**caractérisée en ce que**
deux débitmètres (4, 5) sont disposés l'un derrière l'autre, qui fonctionnent de façon redondante comme débitmètre de fonctionnement (4) et débitmètre de système de protection (5) et sont surveillés par l'ordinateur du dispositif de mélange.

8. Dispositif de mélange selon les revendications 1 à 7,
**caractérisé en ce que**
le contenant de matière première (19) est un contenant à usage unique.

9. Dispositif de mélange selon les revendications 1 à 8,
**caractérisé en ce que**
une lance d'aspiration (20) conduit à la zone inférieure du contenant de matière première (19), par laquelle le mélange de matière première liquide peut être aspiré dans le circuit de recirculation.

10. Dispositif de mélange selon les revendications 1 à 9,
**caractérisé en ce que**
une chambre de dosage (chambre d'équilibrage) (53) est activée dans la conduite de remplissage/évacuation (47) conduisant au contenant (44) de solution prête, du circuit de recirculation.
